Europäisches Patentamt

European Patent Office

Office européen des brevets

(19)

(11) Publication number: **0 241 136**
A2

(12) **EUROPEAN PATENT APPLICATION**

(21) Application number: 87301969.9

(22) Date of filing: 06.03.87

(51) Int. Cl.³: **C 07 K 13/00**
C 07 K 3/02, C 12 N 5/02
A 61 K 37/36

(30) Priority: 07.03.86 US 838096

(43) Date of publication of application:
14.10.87 Bulletin 87/42

(84) Designated Contracting States:
AT BE CH DE FR GB IT LI LU NL SE

(71) Applicant: The President and Fellows of Harvard College
17 Quincy Street
Cambridge, MA 02138(US)

(72) Inventor: Lobb, Roy R.
41 Thurston Road
Newton Upper Falls Massachusetts 02164(US)

(72) Inventor: Harper, J. Wade
41 Hyde Street
Newton Highlands Massachusetts 02161(US)

(72) Inventor: Strydom, Daniel J.
141 Hollingsworth Avenue
Braintree Massachusetts 02184(US)

(74) Representative: Bizley, Richard Edward et al,
BOULT, WADE & TENNANT 27 Furnival Street
London EC4A 1PQ(GB)

(54) Human class 1 heparin-binding growth factor.

(57) A proteinaceous factor which has mitogenic activity comprising a single chain polypeptide of 140 amino acids having a sequence of:

Phe$^1$-Asn-Leu-Pro-Pro-Gly-Asn-Tyr-Lys-Lys-Pro-Lys-Leu-Leu-Tyr$^{15}$-Cys-Ser-Asn-Gly-Gly-His-Phe-Leu-Arg-Ile-Leu-Pro-Asp-Gly-Thr$^{30}$-Val-Asp-Gly-Thr-Arg-Asp-Arg-Ser-Asp-Gln-His-Ile-Gln-Leu-Gln$^{45}$-Leu-Ser-Ala-Glu-Ser-Val-Gly-Glu-Val-Tyr-Ile-Lys-SHand the physiologically active fragments thereof.

This invention further comprises a substantially purified proteinaceous factor obtained from human brain tissue which has mitogenic activity, has a molecular weight of about 15200 to about 16500 and is characterized by having a high affinity for heparin and its purification process.

EP 0 241 136 A2

# HUMAN CLASS 1 HEPARIN-BINDING GROWTH FACTOR

This invention relates to a novel, substantially purified proteinaceous factor obtained from human brain tissue which has mitogenic activity. This invention further relates to its purification and uses.

Heparin-binding growth factors (HBGF's) can be grouped into two classes on the basis of a number of criteria (Lobb et aL (1986) J. BioL Chem. 261, 1924-1928). Class 1 HBGF's are found in high levels in neural tissue, and are typified by bovine acidic brain fibroblast growth factor (FGF) (Thomas et al. (1984) Proc. NatL Acad. Sci. U.S.A. 81, 357-361). Class 2 HBGF's are found in a variety of tissues and are exemplified by bovine pituitary FGF (Gospodarowicz et al. (1984) Proc. Natl. Acad. Sci. U.S.A. 81, 6963-6967) and cartilage-derived growth factor (Sullivan et aL (1985) J. Biol. Chem. 260, 2399-2403). HBGF's are mitogens for a wide variety of mesoderm-derived cells in vitro. In addition, homogeneous class 1 HBGF's from bovine brain induce neovascularization in vivo (Lobb et al. (1985) Biochemistry, 24, 4969-4973; Thomas et al. (1985) Proc. Natl. Acad. Sci. U.S.A. 82, 6409-6413). They are characterized by an unusual affinity for the complex glycosaminoglycan, heparin, and this affinity has been exploited for their purification and characterization from a variety of tissues and species. Class 1 HBGF's have been purified to homogeneity from several neural tissues including brain, hypothalamus, and retina (Conn et al. (1984) Biochem. Biophys. Res. Commun. 124, 262-268; Lobb et al. (1984) Biochemistry, 23, 6925-6929; Thomas et al. (1984) Proc. Natl. Acad. Sci. U.S.A. 81, 357-361; Baird et al. (1985) Biochemistry 24, 7855-7860; Lobb et al. (1985) Biochem. Biophys. Res. Commun. 131, 586-592; Lobb et al. (1986) J. Biol. Chem. 261, 1924-1928). Both the mitogenic and angiogenic activities of class 1 HBGF's are enhanced by heparin (Lobb et al. (1985) Biochemistry, 24, 4969-4973), a characteristic which distinguishes them from class 2 HBGF's.

The amino acid sequences of mitogens typical of both classes, bovine acidic brain FGF (Gimenez-Gallego et al. (1985) Science 230, 1385-1388; Esch et al. (1985) Biochem. Biophys. Res. Commun. 133, 554-562) and bovine pituitary FGF (Esch et al. (1985) Proc. Natl. Acad. Sci. U.S.A. 82, 6507-6511) have recently been completed. The two sequences are distinct, but with about 50% identity of amino acids (Esch et al. (1985) Biochem. Biophys. Res. Commun. 133, 554-562). The human brain-derived class 1 HBGF (HBGF-1) of the present invention is structurally distinguished from bovine brain-derived class 1 HBGF as described herein as Example 13.

0241136

It is therefore an important object of the present invention to provide a purified mitogenic growth factor and active fragments thereof from human brain tissue.

Another object is to provide a process of purifying a human brain-derived mitogen.

A further object is to provide a method for promoting the growth of mesoderm- and neuroectoderm-derived cells.

A still further object is to provide a method for generating endothelial cell linings for vascular prostheses.

Further purposes and objects of the present invention will appear as the specification proceeds.

With the foregoing and other objects in view, the invention herein provides a protein useful as a mitogenic growth factor and a process for the purification of the protein from human brain tissue. The background of the invention and its departure from the art will be further described hereinbelow.

The background of the invention and its departure from the art will be further described hereinbelow with reference to the accompanying drawings, wherein:

FIGURE 1 shows the separation of (A) tryptic peptides and (B) chymotryptic peptides from the human brain heparin-binding growth factor of the present invention by high performance liquid chromatography; and

FIGURE 2 is the alignment of half-cystine residues in human brain class 1 heparin-binding growth factor (HBGF-1) of this invention, bovine brain HBGF-1, and bovine pituitary fibroblast growth factor (FGF or HBGF-2).

The present invention concerns a proteinaceous factor which has mitogenic activity comprising a single chain polypeptide of 140 amino acids having a sequence of:

$Phe^1$-Asn-Leu-Pro-Pro-Gly-Asn-Tyr-Lys-Lys-Pro-Lys-Leu-Leu-$Tyr^{15}$-
Cys-Ser-Asn-Gly-Gly-His-Phe-Leu-Arg-Ile-Leu-Pro-Asp-Gly-$Thr^{30}$-
Val-Asp-Gly-Thr-Arg-Asp-Arg-Ser-Asp-Gln-His-Ile-Gln-Leu-$Gln^{45}$-
Leu-Ser-Ala-Glu-Ser-Val-Gly-Glu-Val-Tyr-Ile-Lys-Ser-Thr-$Glu^{60}$-
Thr-Gly-Gln-Tyr-Leu-Ala-Met-Asp-Thr-Asp-Gly-Leu-Leu-Tyr-$Gly^{75}$-
Ser-Gln-Thr-Pro-Asn-Glu-Glu-Cys-Leu-Phe-Leu-Glu-Arg-Leu-$Glu^{90}$-
Glu-Asn-His-Tyr-Asn-Thr-Tyr-Ile-Ser-Lys-Lys-His-Ala-Glu-$Lys^{105}$-
Asn-Trp-Phe-Val-Gly-Leu-Lys-Lys-Asn-Gly-Ser-Cys-Lys-Arg-$Gly^{120}$-
Pro-Arg-Thr-His-Tyr-Gly-Gln-Lys-Ala-Ile-Leu-Phe-Leu-Pro-$Leu^{135}$-
Pro-Val-Ser-Ser-$Asp^{140}$-OH,

and the physiologically active fragments thereof. The invention further relates to a substantially purified proteinaceous factor obtained from human brain tissue which has mitogenic activity, has a molecular weight of about 15200 to about 16500, typically about 15616 to about 16056, and is characterized by having a high affinity for heparin. The mitogen of this invention differs structurally from acidic brain fibroblast growth factor (FGF), the major class 1 heparin-binding growth factor of bovine brain, by eleven amino acids.

The purification process of this invention involves homogenizing brain tissue and purifying to homogeneity by heparin affinity chromatography in combination with cation exchange and/or reversed-phase high performance liquid chromatography (HPLC). The sequence was solved using fragments generated from both chemical and proteolytic cleavages. The procedures used involved chemical cleavage of the polypeptide chain with cyanogen bromide and hydroxylamine in conjunction with direct Edman degradation as well as proteolytic fragmentation with trypsin and chymotrypsin. Based upon the primary structure of the protein comprising 140 amino acids, the calculated molecular weight is 15836, in good agreement with that determined by sodium dodecyl sulfate-polyacrylamide gel electrophoresis (SDS-PAGE) (Mr of around 17000).

It is to be understood that the present invention is not limited to obtaining the mitogen described herein from human brain tissue. In fact, it is desired and intended to include the polypeptide and the active fragments thereof prepared by any method within the skill of the art such as chemical synthesis or conventional genetic engineering techniques, i.e., gene splicing, gene manipulation, etc. It is further within the contemplation of this invention to include the physiologically active fragments of the polypeptide. Those skilled in the biochemical arts will readily recognize that modification of the polypeptide such as digesting the protein into fragments or preparing synthetic portions of fragments provides fragments which are equally useful as mitogenic growth factors. For example, it is well-known that one or more amino acid residues can be taken off any given protein from the carboxy or amino terminus and the protein will still demonstrate activity. Therefore, the physiologically active fragments of the polypeptide of this invention are within the scope of the present invention.

It is to be further understood that the terms "proteinaceous factor," "substantially purified proteinaceous factor," "human brain-derived class 1 HBGF," "human HBGF-1," "HBGF-1," "human brain-derived mitogen," "mitogenic growth factor," "mitogen," "polypeptide" and "active fragments" are considered equivalents for purposes of describing this invention. Unless otherwise specified, reference to the term "HBGF-1" means human HBGF-1.

The mitogenic growth factor of the instant invention may be found therapeutically and diagnostically useful in a variety of ways. Therapeutic uses for agents that increase vascularization, such as HBGF's, can be found in the following areas: wound healing; myocardial infarction; ischemia (tissue and vessel destruction due to lack of oxygen); and transplantation biology, e.g., skin grafting. Since HBGF's are mitogenic for a wide variety of mesoderm- and neuroectoderm-derived cells, this mitogenic activity may be therapeutically useful in tissue healing in vivo in the absence of neovascularization, e.g., corneal re-endothelialization and stimulation of cell growth in vitro, e.g., in generating endothelial cell linings for vascular prostheses (see Watkins et al. (1984) J. Surg. Res. 36 : 588-596). Increased levels of HBGF's in body fluids may also have diagnostic value in certain neurological diseases. For example, since high levels of HBGF's are found in the brain, patients with brain tumors may develop high levels of HBGF's in cerebrospinal fluid which would be detectable by conventional methods and assays.

For therapeutic uses, a therapeutically effective amount of the mitogen of this invention may be incorporated into an inert, noninflammatory carrier such as methyl cellulose or other compatible pharmaceutically acceptable carrier. The composition desirably contains a carrier which does not significantly or adversely affect the pharmaceutical or physiological properties of the polypeptide such as toxicity, effectiveness, etc. The amount of the polypeptide and the type of pharmaceutical preparation (e.g., ointment, cream, liquid, etc.) will vary according to the needs of the particular therapy. It is apparent to those skilled in the pharmaceutical, medical or biochemical fields how to prepare and use an appropriate pharmaceutical composition containing the mitogenic growth factor for therapeutic purposes. Cell culturing techniques for promoting the growth of cells for therapeutic uses are also well recognized.

The following examples demonstrate certain aspects of the present invention. However, it is to be understood that these examples are for illustrative purposes only and do not purport to be wholly definitive as to conditions and scope of this invention. All parts and percents referred to herein are on a weight basis and all temperatures are expressed in degrees Celsius unless otherwise specified. It also should be appreciated that when typical reaction conditions (e.g., temperature, reaction times) have been given, the conditions which are both above and below these specified ranges can also be used, though generally less conveniently.

A further understanding of the invention may be obtained from the following nonlimiting examples. These examples were conducted at room temperature (about 23°C to about 28°C) and at atmospheric pressure.

## EXAMPLE 1

### Human Brain HBGF-1 Isolation

Human brain tissue was obtained at autopsy and stored at -20°C. Tissue in 600 to 1000 g lots was homogenized, acidified to pH 4.5, and processed through ammonium sulfate precipitation and CM-Sephadex®️ C50 (a gel bead made from dextran and epichlorohydrin, manufactured by Pharmacia Fine Chemicals) cation-exchange chromatography as described previously (Lobb et al. (1985) Biochem. Biophys. Res. Commun. 131, 586-592). Heparin-affinity chromatography was performed by applying the HBGF-containing pool to heparin-Sepharose (Pharmacia) and washing the column with 10 mM Tris·HCl, 0.6 M NaCl, pH 7.0. Subsequently the column was eluted at a flow rate of 10-14 cm/h with 10 mM Tris·HCl, pH 7.0, containing first 0.8 M NaCl, then 1.2 M NaCl, and finally 2.0 M NaCl. The HBGF-1 containing pool (1.2 M NaCl eluate) was subjected to high performance liquid chromatography (HPLC) on either a Mono-S HR5/5 (Pharmacia) cation exchange column or on a propylsilane (C3) column (5 μm particle size, 75 x 4.6 mm, Ultrapore RSPC, Beckman Instruments, Berkeley, CA) (Lobb et al. (1984) Biochemistry, 23, 6925-6929; Lobb et al. (1986) J. Biol. Chem. 261, 1924-1928). Material processed through the Mono-S cation exchange chromatography step was desalted for chemical studies by reversed-phase HPLC on a C3 column as described (Lobb et al. (1984) Biochemistry, 23, 6925-6929). The homogeneous mitogen stimulates half-maximal DNA synthesis in Balb/C 3T3 fibroblasts at a concentration of 250 pg/mL (Lobb et al. (1985) Biochem. Biophys. Res. Commun. 131, 586-592; Lobb et al. (1986) J. Biol. Chem. 261, 1924-1928).

## EXAMPLE 2

### Amino Acid Analysis

Aliquots of proteins and peptides were taken from HPLC fractions and hydrolyzed with 6 N HCl for 18 hours at 100°C. The dried hydrolysates were subjected to amino acid analyses using the "Picotag" method (Waters Picotag columns, Waters Associates) (Bidlingmeyer et al. (1984) J. Chromatogr. 336, 93-104; Cohen et al. (1985) Fed. Proc. 44, 1211). Analyses after performic acid oxidation (Moore (1963) J. Biol. Chem. 238, 235-237) were performed as above except that the HPLC gradient was changed to 5-50% solvent comprising 2-propanol:acetonitrile:water (3:2:2) containing 0.08% w/w trifluoroacetic acid (TFA) in 10 minutes, using a convex gradient (curve 5, Waters Associates gradient controller), which separates $CySO_3H$ and Asp derivatives with baseline resolution ($CySO_3H$, 2.22 min; Asp, 2.50 min; Glu, 2.96 min).

Tryptophan was determined by the standard phenylisothiocyanate (PITC) methodology after hydrolysis with 4N methanesulfonic acid, containing 0.2% 3-(2-aminoethyl) indole (Pierce Chemical Co.) (Liu et al. (1971) J. Biol. Chem. 246, 2842-2848).

## EXAMPLE 3

### Reduction and S-Sulfopropylation

Lyophilized HBGF-1 (4 nmol) was dissolved in 0.4 mL of 0.5 M Tris·HCl, 10 mM EDTA, 6 M guanidine·HCl, pH 7.7. The solution was flushed with $N_2$, dithiothreitol added (final concentration, 6 mM), and reduction allowed to proceed for 45 minutes at 37°C. 1,3-Propane sultone (in 50% 1-propanol, final concentration 40 mM) was added and alkylation allowed to occur for 2 hours at 37°C (Ruegg et al. (1977) Methods Enzymol. 47, 111-116). The alkylated protein was desalted by reversed-phase HPLC (C3).

## EXAMPLE 4

### Proteolytic Digestion

Native HBGF-1 or RS-HBGF-1 (1-9 nmol) as prepared above in Example 3 was dissolved in 0.5 M Tris·HCl, 10 mM EDTA, 6 M guanidine·HCl, pH 7.7, and diluted with 9 volumes of 1% $NaHCO_3$ to give

a final protein concentration of 4.5 nmol/mL. TLCK-treated chymotrypsin (2% w/w chymotrypsin treated with N-α-tosyl-lysine-chloromethyl ketone, Sigma Chemical Co.) or HPLC purified trypsin (5-7% w/w) (Titani et al. (1982) Anal. Biochem. 123, 408-412) was added and the mixture incubated for 21-24 hours at 35°C. The reaction was quenched by addition of 10% TFA (1.3% final concentration).

## EXAMPLE 5

### Peptide Mapping

Proteolytic digests were fractionated on a Synchropak RP-P column (Synchrom, Inc., Linden, IN, 250 x 4.5 mm) employing linear gradients with either a 0.1 M perchloric acid/0.1% phosphoric acid/acetonitrile (pH 2.5) solvent system or a 0.1% TFA/2-propanol/acetonitrile solvent system. Peptides were further purified on an Altex Ultrapore IP column (Beckman Instruments Inc., 250 x 4 mm, 5μm particle size) with 0.1% TFA/acetonitrile solvent systems.

## EXAMPLE 6

### Sequencing Studies

Automated microsequencing was performed with a Beckman Model 890C sequencer updated to System 890 status. The program used was Beckman #347379, Rev 7.8.84, program No. 3, using 0.1M buffer comprising 1,1',1'',1'''-(ethylenedinitrilo) tetra-2-propanol and employing a 5 mg polybrene as carrier. Automated conversion of thiazolinones to phenylthiohydantoins employed 25% TFA containing 0.01% ethanethiol. Phenylthiohydantoin-amino acids (PTH-amino acids) were identified and quantitated according to established procedures (Strydom et al. (1985) Biochemistry 24, 5486-5494).

## EXAMPLE 7

### Cyanogen Bromide Fragmentation

HBGF-1 (1.5 nmol) was applied to the sequencer cup together with 5 mg polybrene and dried. Thirty cycles of Edman degradation were performed. At cycle thirty-one, phenylisocyanate (Aldrich Chem. Co.) was substituted for phenylisothiocyanate. This procedure blocks the open amino terminal end groups (Boosman (1980) "Methods in Peptide and Protein Sequence Analysis", (Birr, C., Ed.) pp. 513-516, Elsevier, New York). Cyanogen bromide (50 mg) was dissolved in 500 μL 70% TFA and added to the sequencer cup at 25°C. After 16 hours the cup was dried and one sequencer cycle, omitting the addition of PITC, was performed to remove remaining reagents and products. Normal sequencer operation was then initiated to sequence the single cyanogen bromide cleavage fragment through twenty cycles.

## EXAMPLE 8

### Hydrazinolysis and Hydroxylamine Fragmentation

Hydrazinolysis and hydroxylamine fragmentation were performed according to established procedures (Strydom et al. (1985) Biochemistry 24, 5486-5494).

## EXAMPLE 9

### N-Terminal Sequencing and Cyanogen Bromide Fragmentation

Table 1 includes the amino acid composition of highly purified human HBGF-1. Table 2 distinguishes the primary structure of human brain HBGF-1 from bovine brain HBGF-1 (Esch et al. (1985) Biochem. Biophys. Res. Commun. 133, 554-562; Gimenez-Gallego et al. (1985) Science 230, 1385-1388).

Edman degradation of human HBGF-1 (1.5 nmol) allowed assignment of the first 31 residues at the N-terminus (see Table 2). Two major sequences were observed; the second sequence begins with a deaminated Asn-2. At cycle 16, the sequencer derived products of cysteine were identified. At cycle 31, phenylisocyanate was substituted for phenylisothiocyanate (Boosman (1980) in "Methods in Peptide and Protein Sequence Analysis" (Birr, C., Ed.) pp. 513-516, Elsevier, New York). This procedure blocks any open end groups, giving a phenylurea derivative which does not undergo Edman degradation. The blocked protein was then subjected to cyanogen bromide cleavage in the sequencer cup at the single methionine residue as described above. The cyanogen bromide fragment with an unblocked α-amino group was then subjected to Edman degradation through 20 cycles (see Table 2).

Table 1.  Amino Acid Compositions of Human Brain HBGF-1 Fragments.[a]

| Peptide: | HC-1 | HC-2 | HC-3 | HC-4 | HC-6b |
|---|---|---|---|---|---|
| Asp | 1.38 | 0.61 | 0.88(1) | | |
| Glu | 3.19(2) | 2.01(2) | 3.01(3) | | 1.14(1) |
| Ser | 2.41(1) | 2.16(2) | 0.40 | | |
| Gly | 4.13(2)[b] | 2.53(1) | 0.47 | | 1.52(1) |
| His | | | 0.94(1) | | |
| Arg | 0.69 | | 0.94(1) | | |
| Thr | 1.72(2) | | | | |
| Ala | 1.20 | 1.42(1) | | | 1.13(2) |
| Pro | 0.37 | 0.35 | | 1.23(1) | |
| Tyr | 1.79(1) | 1.00(1) | 0.73(1) | 0.91(1) | |
| Val | 0.53 | 1.95(2) | | | |
| Met | | | | | |
| Ile | 0.95(1) | | | | 0.84(1) |
| Leu | 1.17 | | 1.06(1) | 2.29(2) | 0.99(1) |
| Phe | 0.36 | | | | 1.04(1) |
| Trp | | | | | |
| Lys | 1.03(1) | | | 2.73(3) | 0.72(1) |
| Cys[*] | | 0[g] | | | |
| pmol analyzed | 45 | 25 | 15 | 29 | 25 |
| sequence position | 56-64 | 47-55 | 87-94 | 9-15 | 126-132 |

Table 1. Amino Acid Compositions of Human Brain HBGF-1
Fragments.[a]

| Peptide: | HC-1 | HC-2 | HC-3 | HC-4 | HC-6b |
|---|---|---|---|---|---|
| Asp | 1.38 | 0.61 | 0.88(1) | | |
| Glu | 3.19(2) | 2.01(2) | 3.01(3) | | 1.14(1) |
| Ser | 2.41(1) | 2.16(2) | 0.40 | | |
| Gly | 4.13(2)[b] | 2.53(1) | 0.47 | | 1.52(1) |
| His | | | 0.94(1) | | |
| Arg | 0.69 | | 0.94(1) | | |
| Thr | 1.72(2) | | | | |
| Ala | 1.20 | 1.42(1) | | | 1.13(1) |
| Pro | 0.37 | 0.35 | | 1.23(1) | |
| Tyr | 1.79(1) | 1.00(1) | 0.73(1) | 0.91(1) | |
| Val | 0.53 | 1.95(2) | | | |
| Met | | | | | |
| Ile | 0.95(1) | | | | 0.84(1) |
| Leu | 1.17 | | 1.06(1) | 2.29(2) | 0.99(1) |
| Phe | 0.36 | | | | 1.04(1) |
| Trp | | | | | |
| Lys | 1.03(1) | | | 2.73(3) | 0.72(1) |
| Cys[e] | | 0[g] | | | |
| pmol analyzed | 45 | 25 | 15 | 29 | 25 |
| sequence position | 56-64 | 47-55 | 87-94 | 9-15 | 126-132 |

Table 1. (cont.)

| Peptide: | HC-8 | HC-10a | HC-10b | HC-11 | HC-13[c] |
|---|---|---|---|---|---|
| Asp | 1.09(1) | 1.32(1) | 1.05(1) | 1.83(2) | 4.36(4) |
| Glu | | 3.29(3) | 1.46(1) | 0.23 | 2.47(3) |
| Ser | 1.94(2) | 0.48 | 1.38(1) | 0.46 | 1.74(1) |
| Gly | 0.32 | 0.84 | 0.59 | 1.45(1) | 3.42(2) |
| His | | 0.96(1) | 1.10(1) | 0.10 | 0.81(1) |
| Arg | | 0.83(1) | | | 3.10(3) |
| Thr | | | | 0.89(1) | 2.36(2) |
| Ala | | 0.28 | 1.20(1) | 1.25(1) | 0.51 |
| Pro | 2.67(2) | 0.27 | | | 1.85(1) |
| Tyr | | 1.06(1) | | 0.97(1) | |
| Val | 0.97(1) | | | | 0.98(1) |
| Met | | | | 0.81(1) | |
| Ile | | | 1.07(1) | | 2.04(2) |
| Leu | 2.05(2) | 2.21(2) | | 2.87(3) | 3.81(4) |
| Phe | 0.90(1) | 0.98(1) | 0.93(1) | | 0.37 |
| Trp | | | (1)[h] | | |
| Lys | | | 2.60(3) | | |
| Cys[e] | | | | | |
| pmoles analyzed | 25 | 30 | 25 | 30 | 20 |
| sequence position | 132-140 | 85-94 | 98-108 | 65-74 | 23-46 |

Table 1. (cont.)

| Peptide: | HT-1b | HT-3 | HT-4[c] | HT-8a | HT-8b |
|---|---|---|---|---|---|
| Asp | . | 2.13(2) | 2.03(2) | 0.91(1) | 0.89(1) |
| Glu | 0.99(1) | 0.65 | 2.24(2) | 0.15 | 1.01(1) |
| Ser | 0.18 | 0.75 | 2.07(1) | 1.18(1) | 0.52 |
| Gly | 1.13(1) | 3.0(2) | 1.85 | 2.27(2) | 1.49(1) |
| His | 0.90(1) | | 0.94(1) | 0.84(1) | 0.73(1) |
| Arg | | 1.07(1) | 0.33 | 0.98(1) | |
| Thr | 0.98(1) | 1.95(2) | 1.26(1) | | |
| Ala | | 0.39 | 0.50 | | 1.02(1) |
| Pro | | 1.11(1) | 0.39 | | |
| Tyr | 0.97(1) | 0.31 | 2.09(2) | 1.29(1) | |
| Val | | 1.19(1) | | | 0.94(1) |
| Met | | | | | |
| Ile | | 1.09(1) | 1.22(1) | | |
| Leu | | 1.35(1) | 1.48(1) | 3.04(3) | 1.03(1) |
| Phe | | 0.18 | — | 1.05(1) | 0.89(1) |
| Trp | | | | | (1) |
| Lys | 0.98(1) | 0.39 | 1.99(2) | | 2.67(3) |
| Cys[e] | | | | 0.73(1) | |
| pmoles analyzed | 60 | 50 | 20 | 30 | 30 |
| sequence position | 123-128 | 25-35 | 89-101 | 13-24 | 102-113 |

Table 1. (cont.)

| Peptide: | HT-13 | HT-15 | HT-18b |
|---|---|---|---|
| Asp | 1.91(2) | 1.17(1) | 5.17(5) |
| Glu | 4.01(5) | 0.27 | 8.20(8) |
| Ser | 3.08(3) | 2.09(2) | 4.00(3) |
| Gly | 2.76(1) | 0.69 | 4.37(3) |
| His | 0.64(1) | | 0.91(1) |
| Arg | 0.94(1) | | 1.14(1) |
| Thr | 0.34 | | 4.96(5) |
| Ala | 1.05(1) | 1.11(1) | 1.18(1) |
| Pro | 0.19 | 1.99(2) | 1.30(1) |
| Tyr | 1.06(1) | | 4.60(4) |
| Val | 1.57(2) | 1.13(1) | 0.33 |
| Met | | | 1.33(1) |
| Ile | 1.43(2) | 1.03(1) | 1.17(1) |
| Leu | 1.83(2) | 2.99(3) | 6.28(6) |
| Phe | 0.30 | 1.08(1) | 1.39(1) |
| Trp | | | |
| Lys | 1.15(1) | | 2.11(2) |
| Cys[a] | | | 0.54(1) |
| pmoles analyzed | 32 | 65 | 7.5 |
| sequence position | 36-57 | 129-140 | 58-101 |

Table 1. (cont.)

| Peptide: | HY-2 | Human brain HBGF-1 | |
|---|---|---|---|
| | | from sequence[d] | from amino acid analysis |
| Asp | 1.30(1) | 15 | 14.3 |
| Glu | 1.37(1) | 15 | 15.9 |
| Ser | 2.63(3) | 10 | 9.4 |
| Gly | 3.25(3) | 13 | 14.2 |
| His | 0.65(1) | 5 | 4.7 |
| Arg | 1.65(2) | 6 | 6.0 |
| Thr | 0.95(1) | 8 | 7.9 |
| Ala | 1.17(1) | 4 | 4.4 |
| Pro | 2.46(3) | 8 | 8.3 |
| Tyr | ---(1)[j] | 8 | 7.3 |
| Val | 1.24(1) | 5 | 4.7 |
| Met | | 1 | 0.8 |
| Ile | 1.03(1) | 5 | 4.1 |
| Leu | 2.80(3) | 17 | 16.9 |
| Phe | 1.02(1) | 5 | 4.0 |
| Trp | | 1 | (1)[g] |
| Lys | 1.79(2) | 11 | 11.4 |
| Cys[e] | 0.80(1)[f] | 3 | 2.6[f,i] |
| pmoles analyzed | 33 | | |
| sequence position | 115-140 | Total = 140 | |

Footnotes to Table 1.

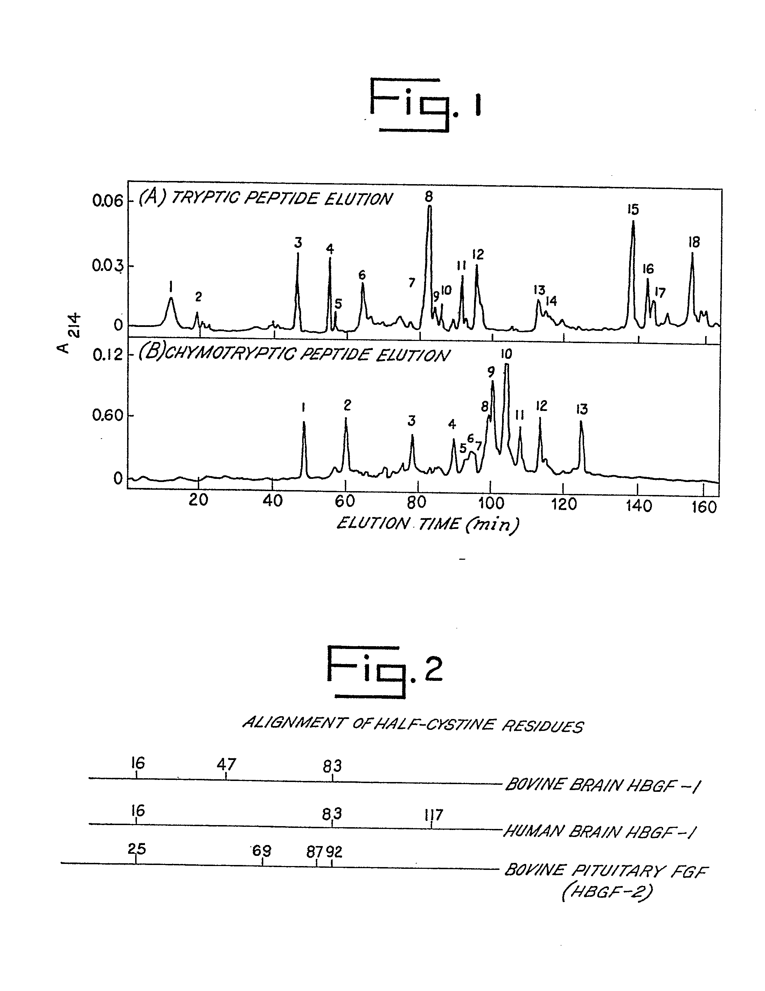

[a]Relative molar amounts of amino acids are given. Chymotryptic (HC) and tryptic (HT) peptides are numbered according to their HPLC elution order (see Figure2). The C-terminal hydroxylamine cleavage fragment is designated HY-2. Unless otherwise noted, peptides were rechromatographed using volatile buffers prior to amino acid analysis. Numbers in parentheses indicate the expected number of residues from sequence.

[b]Analyses are not corrected for Gly, Ser, Ala, Glu, and Asp, which are present at this level in some of the HPLC fractions.

[c]Analysis performed on aliquot from initial HPLC fractionation.

[d]See Figure 1.

[e]Determined as S-sulfopropylcysteine unless otherwise noted.

[f]Determined as cysteic acid after performic acid oxidation.

[g]No trace of cysteic acid found after performic acid oxidation.

[h]Determined from Edman degradation of HC-10b.

[i]Average of two determinations with 27-30 pmoles of protein analyzed.

[j]Performic acid oxidation for detection of cysteine results in destruction of tyrosine.

## TABLE 2 - PRIMARY STRUCTURE OF HUMAN BRAIN HBGF-1 AND BOVINE BRAIN HBGF-1[a]

```
                                        10                              20                          30
Bovine  Phe-Asn-Leu-Pro-[Leu]-Gly-Asn-Tyr-Lys-Lys-Pro-Lys-Leu-Leu-Tyr-Cys-Ser-Asn-Gly-Gly-[Tyr]-Phe-Leu-Arg-Ile-Leu-Pro-Asp-Gly-Thr-
Human   Phe-Asn-Leu-Pro-[Pro]-Gly-Asn-Tyr-Lys-Lys-Pro-Lys-Leu-Leu-Tyr-Cys-Ser-Asn-Gly-Gly-[His]-Phe-Leu-Arg-Ile-Leu-Pro-Asp-Gly-Thr-
                             ED
                                                                          HY-3
                        ..........HC-4...........                ................HC-13...........
                                ...............HT-8a................      HT-3
```

```
                                        40                              50                          60
Bovine  Val-Asp-Gly-Thr-[Lys]-Asp-Arg-Ser-Asp-Gln-His-Ile-Gln-Leu-Gln-Leu-[Cys]-Ala-Glu-Ser-[Ile]-Gly-Glu-Val-Tyr-Ile-Lys-Ser-Thr-Glu-
Human   Val-Asp-Gly-Thr-[Arg]-Asp-Arg-Ser-Asp-Gln-His-Ile-Gln-Leu-Gln-Leu-[Ser]-Ala-Glu-Ser-[Val]-Gly-Glu-Val-Tyr-Ile-Lys-Ser-Thr-Glu-

                     ....................HC-13....................       HC-2                        HC-1
        ........HT-3.........                                                                        ..HT-18b...
                                            HT-13
```

```
                                        70                              80                          90
Bovine  Thr-Gly-Gln-[Phe]-Leu-Ala-Met-Asp-Thr-Asp-Gly-Leu-Leu-Tyr-Gly-Ser-Gln-Thr-Pro-Asn-Glu-Glu-Cys-Leu-Phe-Leu-Glu-Arg-Leu-Glu-
Human   Thr-Gly-Gln-[Tyr]-Leu-Ala-Met-Asp-Thr-Asp-Gly-Leu-Leu-Tyr-Gly-Ser-Gln-Thr-Pro-Asn-Glu-Glu-Cys-Leu-Phe-Leu-Glu-Arg-Leu-Glu-
                                            CNBR-2                                                   HC-10a
            HC-1            HC-11                                                                    ......HC-3.....
        ...................................................HT-18b........................
                                                                                                    HT-4
```

```
                                        100                             110                         120
Bovine  Glu-Asn-His-Tyr-Asn-Thr-Tyr-Ile-Ser-Lys-Lys-His-Ala-Glu-Lys-[His]-Trp-Phe-Val-Gly-Leu-Lys-Lys-Asn-Gly-[Arg-Ser]-Lys-[Leu]-Gly-
Human   Glu-Asn-His-Tyr-Asn-Thr-Tyr-Ile-Ser-Lys-Lys-His-Ala-Glu-Lys-[Asn]-Trp-Phe-Val-Gly-Leu-Lys-Lys-Asn-Gly-[Ser-Cys]-Lys-[Arg]-Gly-
            HC-10a                                            HY-2
        .....HC-3......                   HC-10b
        .............HT-18b...................
                        HT-4
                                                            HT-8b
```

```
                                        130                             140
Bovine  Pro-Arg-Thr-His-[Phe]-Gly-Gln-Lys-Ala-Ile-Leu-Phe-Leu-Pro-Leu-Pro-Val-Ser-Ser-Asp
Human   Pro-Arg-Thr-His-[Tyr]-Gly-Gln-Lys-Ala-Ile-Leu-Phe-Leu-Pro-Leu-Pro-Val-Ser-Ser-Asp
                                HY-2
                        ..........HC-6b............
                                                            HC-8
        .......HT-1b...........  ..................HT-15....................
```

Footnote to Table 2.

[a]The extent of Edman degradation of cyanogen bromide (CNBr), hydro-xylamine (HY), chymotryptic (HC), and tryptic (HT) digest products and of the intact molecule (ED) is indicated by solid lines. Dashed lines indicate tentative assignments with dotted lines indicating placement by amino acid composition. Gaps indicate where no assignment was made. Dotted lines alone denote sequences confirmed by amino acid composition of peptides. Chymotryptic and tryptic peptides are numbered according to their HPLC elution order (see Figure 2). Boxed regions indicate non-identical residues.

Table 3. Amino Acid Sequences of Some Chymotryptic and Tryptic
Peptides of Human HBGF-1 Established by Edman Degradation.

| Peptide | Sequencer Results[a] |
|---|---|
| HC-1[b] | Ile-Lys-Ser-Thr-Glu-Thr-Gly-Gln-Tyr<br>324 200 241 26 146 28 178 121 197 |
| HC-2 | Ser-Ala-Glu-Ser-Val-Gly-Glu-Val-Tyr<br>233 541 296 91 407 228 330 317 197 |
| HC-8 | Phe-Leu-Pro-Leu-Pro-Val-Ser-Ser-Asp<br>342 361 146 275 92 256 15 130 70 |
| HC-10a | Phe-Leu-Glu-Arg-Leu-Glu-Glu-Asn-His-Tyr<br>100 68 89 59 65 54 70 41 (+) 44 |
| HC-10b | Ile-Ser-Lys-Lys-His-Ala-Glu-Lys-Asn-Trp-Phe<br>221 45 23 127 50 86 77 63 64 6 12 |
| HC-11 | Leu-Ala-Met-Asp-Thr-Asp-Gly-Leu-Leu-Tyr<br>214 154 71 116 (+) 47 105 43 61 80 |
| HT-3 | Ile-Leu-Pro-Asp-Gly-Thr-Val-Asp-Gly-Thr-Arg<br>107 195 80 116 91 (+) 130 57 142 (+) 15 |
| HT-4 | Leu-Glu-Glu-Asn-His-Tyr-Asn-Thr-Tyr-Ile-Ser-Lys-Lys<br>213 79 82 29 — 53 25 (+) 33 35 — 15 13 |
| HT-8b | His-Ala-Glu-Lys-Asn-Trp-Phe-Val-Gly-Leu-Lys-Lys<br>48 277 316 106 160 5: 62 94 70 87 60 40 |
| HT-13 | Asp-Arg-Ser-Asp-Gln-His-Ile-Gln-Leu-Gln-<br>197 56 22 79 34 (?) 24 49 59 42 |
|  | Leu-Ser-Ala-Gln-Ser-Val-Gly-Gly-Val-Tyr<br>42 (+) 21 27 — 11 16 — — 6 |

[a] The established sequence is given, with the yield of the
PTH-amino acid at each cycle given below the residue, in
picomoles.

[b] No trace of Leu found at cycle 10.

## EXAMPLE 10

### Hydroxylamine Fragmentation

Human HBGF-1 (2 nmol) was digested with 2 M hydroxylamine (pH 9), the fragments fractionated by molecular sieve chromatography (I-125 HPLC gel filtration column, Waters Associates), and the lower molecular weight peptides isolated by reversed-phase HPLC. The sequence of a low molecular weight peptide, HY-2, was determined through 18 cycles (see Table 2). At cycle three, the sequencer derived products of cysteine were identified. Consistent with this assignment, amino acid analysis of HY-2 after performic acid oxidation showed the presence of 0.8 residues of cysteic acid (Table 1).

The higher molecular weight fragment obtained from molecular sieve chromatography was sequenced through twelve cycles and showed the presence of two major sequences, one beginning at Gly-19 (HY-3, Table 2) and the other beginning at the amino terminal (Phe-1). These results suggest the presence of a disulfide bridge from Cys-16 to a half-cystine residue in HY-3. Consistent with the N-terminal micro-heterogeneity discussed above, a minor sequence beginning with a deaminated Asn-2 was also observed.

## EXAMPLE 11

### Proteolytic Fragmentation

Peptides generated from tryptic and chymotryptic digests of either reduced and S-sulfopropylated or native human HBGF-1 were mapped by reversed-phase HPLC as shown in Figure 1. Map (A) illustrates separation of tryptic peptides from human brain RS-HBGF-1 by HPLC on a Synchropak RP-P (C18) column. RS-HBGF-1 (1.5 nmol) was incubated with trypsin for 21 hours at 35°C and the reaction quenched with 10% TFA (1.3% final concentration) before fractionation by HPLC. Peptide elution was accomplished by applying HBGF-1 to the column using 0.1% TFA and adding a second solvent 2-propanol:acetonitrile:water (3:2:2) containing 0.08% TFA to form a linear gradient of 0% to 50% of the second solvent in 3 hours at a flow rate of 0.8 mL/min. The fractions that contained significant quantities of amino acids after hydrolysis are numbered according to their order of elution. Map (B) illustrates separation of chymotryptic peptides from human brain HBGF-1 by HPLC on a Synchropak RP-P (C18) column. HBGF-1 (7 nmoles) was digested with chymotrypsin for 21 hours at 35°C and the reaction quenched with 10% TFA (1.3% final concentration). HBGF-1 was applied to the column using 0.1 M perchloric acid/0.1% phosphoric acid (pH 2.5). Peptides were eluted with a linear gradient from 0% to 50% solvent comprising 75% acetonitrile and 25% 0.1 M perchloric acid/0.1% phosphoric acid (pH 2.5) in 3 hours at a flow rate of 0.8 mL/min.

The amino acid composition of some of the peptides obtained in pure form are given in Table 1. The sequences of most of these peptides were determined by Edman degradation (see Tables 2 and 3).

## EXAMPLE 12

### C-Terminal Analysis

Micro-hydrazinolysis on 200 pmoles of the tryptic peptide (HT-15) lacking Lys and Arg established the C-terminal residue as aspartic acid (see Table 2). Consistent with this assignment, the sequence of the chymotryptic peptide HC-8 (see Tables 2 and 3) also terminated with aspartic acid.

The presence of a single methionine residue in human HBGF-1 (Table 1) provided an opportunity for sequencing of the intact protein and cyanogen bromide fragment in a single experiment. For HBGF-1, this strategy allowed the determination of both the amino terminal 31 residues and the sequence of the 20 residues following the single methionine. Thus, 36% of the sequence was established in one experiment utilizing only 1.5 nmoles of human HBGF-1.

Sequencing of fragments generated by hydroxylamine cleavage of the HBGF-1 polypeptide chain confirmed the assignment of residues 19 to 29 at the amino terminus and also revealed the presence of a second Asn-Gly bond between residues 114 and 115. Asn-Gly bonds at positions 18-19 and 114-115 are also found in bovine brain HBGF-1 (see Table 2). The sequence of the hydroxylamine fragment HY-2 derived from cleavage at this second Asn-Gly bond was established by its amino terminal sequence and the sequence of the chymotryptic peptide HC-8. The tryptic peptide HT-15 provided a compositional overlap. Micro-hydrazinolysis of tryptic peptide HT-15 established aspartic acid as the C-terminal amino acid of human HBGF-1, which is identical to that found with bovine HBGF-1 (Gimenez-Gallego et al. (1985) Science 230, 1385-1388; Esch et al. (1985) Proc. Natl. Acad. Sci. U.S.A. 82, 6507-6511). Residue 114 was assigned as asparagine based on the strict specificity of hydroxylamine cleavage only at Asn-Xxx bonds (Bornstein (1969) Biochem. Biophys. Res. Commun. 36, 957; Bornstein et al. (1977) Methods Enzymol. 47, 132-145; Blodgett et al. (1985) J. Amer. Chem. Soc. 107, 4305-4313).

Proteolytic cleavage of human HBGF-1 with either chymotrypsin or trypsin generated short, easily characterized fragments. The sequences of peptides HT-3, HT-13, HC-2, HC-1, HC-11, HC-10a, HT-4, HC-10b, and HT-8b (see Tables 1 and 2) provided the unknown regions of the molecule and confirmed portions of the sequence established using chemical cleavage fragments. The amino acid compositions of peptides HC-13, HT-18b, HT-15, and HC-6b illustrated some overlaps with the sequence of bovine HBGF-1 (see Table 2).

In summary, the data allowed for the description of the sequence of human brain HBGF-1 from residues 1 through 113 and from residues 115 through 140, with residue 114 assigned as asparagine. As such, an intervening amino acid(s) between Lys-113 and Asn-114 is contemplated as within the scope of this invention. However, the close similarity of the amino acid composition of HBGF-1 determined by amino acid analysis with that calculated from the sequence (Table 1), in combination with the comparison to bovine brain HBGF-1, strongly suggests that the alignment shown in Table 2 represents the complete sequence of human brain HBGF-1.

## EXAMPLE 13

### Comparison of Primary Structure of
### Human Brain HBGF-1 and Bovine Brain HBGF-1

The primary structures of both human and bovine brain HBGF-1, which shows 92% identity, are compared in Table 2. Two regions in bovine brain HBGF-1 containing clusters of basic residues (Lys-9 to Lys-12 and Lys-100 to Arg-122), represent likely heparin-binding sites. Residues 9-12 are fully conserved between the human and bovine proteins, while in the region from 100 to 122, four substitutions occur. The highly acidic cluster found between residues 80 and 91 of human HBGF-1 is fully conserved in the bovine protein.

Eleven amino acids differ between human and bovine HBGF-1 (Table 2). Eight of these changes (positions 21, 35, 47, 51, 64, 106, 117, 125) are conservative, resulting in an overall sequence similarity of 98%. The most striking difference between human and bovine HBGF-1 relates to the placement of the three half-cystine residues. Surprisingly, exchanges of cysteine for serine and serine for cysteine have occurred at positions 47 and 117, respectively (see Table 2). Thus, while both proteins contain three half-cystine residues, only two of the three are conserved (positions 16 and 83). The existence of a disulfide bond between Cys-16 and Cys-83 has been suggested in bovine HBGF-1. With human HBGF-1, hydroxylamine fragmentation indeed suggests that a disulfide bond exists between Cys-16 and Cys-83. Moreover, the isolation of the cysteine containing peptide HY-2 from the hydroxylamine fragmentation digest of human HBGF-1 suggests that Cys-117 exists as the free sulfhydryl. Figure 2 shows the alignment of these half-cystine residues in human brain HBGF-1 and bovine brain HBGF-1, and compares them with the protein bovine pituitary FGF, a class 2 HBGF. The half-cystines at positions 16 and 83 in the human brain and bovine brain class 1 HBGF's are also conserved in bovine pituitary FGF at positions 25 and 92, respectively.

Since cysteinyl residues are quite rare in proteins, the Cys/Ser interchanges observed suggest a specific role for a cysteinyl residue in the function of these mitogens. While the cysteinyl residues in bovine and human HBGF-1 are found in different positions in the peptide chain, the tertiary structure of the protein may place these residues in a similar critical spatial arrangement. Alternatively, the role of the cysteinyl residue may be as a carrier of a small molecule of importance for mitogenic activity. In the case of bovine pituitary FGF, two of the four half-cystine residues show evidence of linkage via disulfides to a small molecule (Esch et al. (1985) Proc. Natl. Acad. Sci. U.S.A. 82, 6507-6511). This interaction may be with free cysteine or with a cysteine-containing molecule such as glutathione.

Human HBGF-1 exhibits N-terminal microheterogeneity. The N-terminal sequence from three preparations of human HBGF-1 each yielded two sequences in approximately equal quantities, the second beginning with a deaminated Asn-2. This differs from the N-terminal microheterogeneity exhibited by bovine brain HBGF-1 (Thomas et al. (1985) Proc. Natl. Acad. Sci. U.S.A. 82, 6409-6413) where a form beginning with Asn-7 is obtained in varying amounts ranging from 40-60%. Moreover, bovine pituitary FGF also occurs as a microheterogeneous form beginning with His-16 (Esch et al. (1985) Biochem. Biophys. Res. Commun. 133, 554-562), the position homologous to Asn-7 in bovine HBGF-1. In human HBGF-1, it is possible that Pro-5 restricts proteolytic cleavage at the (Gly-6)-(Asn-7) bond.

A further difference between human and bovine HBGF-1 involves the presence of a potential glycosylation site in the human protein (Asn-Gly-Ser, residues 114-116) which, due to the Arg/Ser interchange at position 116, is not found in bovine HBGF-1.

In the foregoing examples, there has been provided a detailed description of preferred embodiments of the present invention for the purpose of illustration and not limitation. It is to be understood that all other modifications, ramifications and equivalents obvious to those having skill in the art based on this disclosure are intended to be within the scope of the invention as claimed.

CLAIMS:

1. A substantially purified proteinaceous factor obtained from human brain tissue which has mitogenic activity, has a molecular weight of about 15200 to about 16500 and has a high affinity for heparin.

2. The proteinaceous factor according to Claim 1, wherein said molecular weight is about 15616 to about 16056.

3. A proteinaceous factor which has mitogenic activity comprising a single chain polypeptide of 140 amino acids having a sequence of:
$Phe^1$-Asn-Leu-Pro-Pro-Gly-Asn-Tyr-Lys-Lys-Pro-Lys-Leu-Leu-$Tyr^{15}$-Cys-Ser-Asn-Gly-Gly-His-Phe-Leu-Arg-Ile-Leu-Pro-Asp-Gly-$Thr^{30}$-Val-Asp-Gly-Thr-Arg-Asp-Arg-Ser-Asp-Gln-His-Ile-Gln-Leu-$Gln^{45}$-Leu-Ser-Ala-Glu-Ser-Val-Gly-Glu-Val-Tyr-Ile-Lys-Ser-Thr-$Glu^{60}$-Thr-Gly-Gln-Tyr-Leu-Ala-Met-Asp-Thr-Asp-Gly-Leu-Leu-Tyr-$Gly^{75}$-Ser-Gln-Thr-Pro-Asn-Glu-Glu-Cys-Leu-Phe-Leu-Glu-Arg-Leu-$Glu^{90}$-Glu-Asn-His-Tyr-Asn-Thr-Tyr-Ile-Ser-Lys-Lys-His-Ala-Glu-$Lys^{105}$-Asn-Trp-Phe-Val-Gly-Leu-Lys-Lys-Asn-Gly-Ser-Cys-Lys-Arg-$Gly^{120}$-Pro-Arg-Thr-His-Tyr-Gly-Gln-Lys-Ala-Ile-Leu-Phe-Leu-Pro-$Leu^{135}$-Pro-Val-Ser-Ser-$Asp^{140}$-OH.

4. A fragment of the polypeptide of Claim 3 having mitogenic activity.

5. The polypeptide according to Claim 3, wherein said polypeptide comprises a substantially purified polypeptide obtained from human brain tissue.

6. The fragment according to Claim 4, wherein said fragment is obtained from human brain tissue.

7. A process of purifying a proteinaceous class 1 heparin-binding growth factor from brain tissue which comprises homogenizing brain tissue, precipitating a solid, subjecting said solid to heparin affinity chromatography, extracting an eluate and subjecting said eluate to high performance liquid chromatography.

8. A process of purifying a proteinaceous class 1 heparin-binding growth factor from brain tissue which comprises homogenizing brain tissue, precipitating a solid, subjecting said solid to cation exchange chromatography, extracting a first eluate, subjecting said first eluate to heparin affinity chromatography, extracting a second eluate and subjecting said second eluate to high performance liquid chromatography.

9. The process according to Claim 7 or 8, wherein said high performance liquid chromatography is performed on a cation exchange column or a propylsilane column.

10. The process according to claim 7 or 8, wherein said step involving high performance liquid chromatography comprises reversed-phase high performance liquid chromatography, optionally preceded by cation exchange chromatography.

11. A method of promoting the growth of mesoderm-derived cells or neuroectoderm-derived cells which comprises contacting said cells with the polypeptide of claim 3 or the fragment of claim 4.

12. A method of generating endothelial cell linings for vascular prostheses which comprises contacting endothelial cells with the polypeptide of claim 3 or the fragment of claim 4.

13. The use of a factor of any one of claims 1 to 3 or 5 or of a fragment as claimed in claim 4 or claim 6 in preparing a medicament.

# Fig.1

**(A) TRYPTIC PEPTIDE ELUTION**

**(B) CHYMOTRYPTIC PEPTIDE ELUTION**

ELUTION TIME (min)

# Fig.2

ALIGNMENT OF HALF-CYSTINE RESIDUES

BOVINE BRAIN HBGF –1

HUMAN BRAIN HBGF –1

BOVINE PITUITARY FGF (HBGF–2)